(19) Europäisches Patentamt / European Patent Office / Office européen des brevets

(11) EP 3 769 666 A1

(12) **EUROPEAN PATENT APPLICATION**
published in accordance with Art. 153(4) EPC

(43) Date of publication:
27.01.2021 Bulletin 2021/04

(21) Application number: 19771098.1

(22) Date of filing: 27.02.2019

(51) Int Cl.:
A61B 5/00 (2006.01)     A61B 5/021 (2006.01)
A61B 5/145 (2006.01)    A61B 5/0402 (2006.01)
A61B 5/01 (2006.01)     A61B 5/11 (2006.01)

(86) International application number:
PCT/KR2019/002348

(87) International publication number:
WO 2019/182258 (26.09.2019 Gazette 2019/39)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 21.03.2018 KR 20180032868

(71) Applicant: Biomedilabs Co., Ltd.
Seoul 06542 (KR)

(72) Inventor: KIM, Doug Won
Seoul 01690 (KR)

(74) Representative: Lichti - Patentanwälte
Partnerschaft mbB
Postfach 41 07 60
76207 Karlsruhe (DE)

(54) **HUMAN BODY WEARABLE BODY INFORMATION MEASURING DEVICE AND MEDICAL SUPPORT SYSTEM USING SAME**

(57)     Disclosed are a human body wearable body information measuring device and a medical support system based on same, the device comprising: multiple light sources capable of emitting beams having multiple different wavelengths; a photodiode for receiving beams, which are emitted by the multiple light sources and then reflected and scattered by a body portion, and converting the received beams into electrical signals; a blood glucose analysing module for analysing electrical signals to obtain the signal amount of a pulse wave type signal corresponding to each of the wavelengths, and calculating the blood glucose level of a subject to be measured, by using a difference signal amount between a pre-input reference value and the signal amount obtained from the body of the subject to be measured; a blood pressure analysing module for analysing a pulse wave to distinguish between an incident wave and a reflective wave, detecting the PRT and the pulse pressure, and deriving a blood pressure value through a predetermined calculation procedure; and a communication device capable of transmitting, to the outside, the blood glucose and blood pressure values obtained by the modules.

Fig. 3

Description

Technical Field:

The present invention relates to a human body wearable body information measuring device and a medical support system based on the same, and more particularly, to a human body wearable body information measuring device and a medical support system based on the same, which can measure body information including blood pressure and blood glucose which was hard to measure substantially in a non-invasive manner, continuously observe a wearer's conditions requiring attention, and transfer measured data in emergencies so that a person or an organization related with the wearer can do relief work or medical aid.

Background Art:

The average life of humans is increasing at a rapid speed due to development of industrial economy and development of medical technology and a hygienic sense, and the aging population accelerates at a rapid speed. With enhancement of living standard and extension of desired lifetime, interest in health is also increasing. Depending on the increase of interest in health, goods and service industries related with healthcare industry have developed rapidly.
Goods related with healthcare industry have been widely spread. For instance, there are electronic digital thermometers, pulsimeters, blood pressure gauges, low frequency therapeutic apparatuses, self-blood glucose monitoring devices, self-body type diagnosis and reshaping programs mounted in various healthcare machines, and so on.
Such healthcare-related goods are generally distributed as independent products, but human body wearable products allowing a wearer to engage in freely physical activities except for simple measuring devices for measuring pulse, a breathing rate, body temperature, and so on are not yet developed enough to satisfy sufficiently.
In the meantime, due to development of industry, the economic status is improved and physical activity level is reduced. However, people eat a great deal of meat and high calorific food which was hard to eat in the past, but it causes an increase of incidence of diabetes and cardiovascular disease and also causes a high death rate.
The diabetes and cardiovascular disease do not usually accompany severe pains of an affected part, such as external injury or stomach ache, or decrease in physical performance enough to have mobility difficulties in an onset stage, are developing slowly by living habits, especially, eating habits, and patients may not feel symptoms of the disease deeply. Moreover, even after undergoing a medical examination due to physical symptoms, the patients need continuous and careful management.
For instance, cardiovascular disease (CVD) is a major cause of death together with stroke, the number of deaths by CVD is increasing, but it is difficult to forecast it and people spend heavy charges for health care for disease prevention and treatment.
Hypertension is considered as the most important risk factor of cardiovascular disease and stroke, and is very influenced by the blood pressure level. A great deal of studies and clinical outcomes revealed that the absolute value of blood pressure and BP variability (BPV) are important factors of the symptoms of the diseases.
Therefore, it is considered to be very important to minimize risks by continuously monitoring BPV and control risk factors at an early stage, so it is being requested to continuously measure blood pressure of a patient who requires attention.
However, diabetes and cardiovascular disease are not often managed continuously and carefully for a variety of reasons.
Furthermore, with the aging population and the change in social culture, cases that an elderly person or an old couple live in each house alone without living with their descendants and a single person or an unmarried person lives in each house alone are increasing. In those cases, it is difficult to effectively and rapidly cope with emergencies when they are in shock or are indisposed due to an emergency, such as the outbreak of an urgent disease, in their houses.
Therefore, people individually and socially request a measuring device and a support system, which can prevent occurrence of a disease or a serious turn of the disease through a continuous monitoring and steady management to the symptoms of diseases and physical conditions of patients, rapidly sense the patient's emergency so that the patient can receive first aid and a medical organization related with the patient can give a proper treatment.
Additionally, there are lots of cases that indisposed patients or the aged frequently or periodically undergo medical examinations on their diseases through out-clinic services without hospitalization, but in most cases, simple measurements are carried out and it is difficult to measure the conditions of the patient, which may be changed easily, just with the measurement data. Such a situation is very uncomfortable to patients or the aged, is less-effective, and causes lots of expenses socially. Therefore, it is preferable to be equipped with a measuring device and a support system, which can provide the basic telemedicine services to reduce such inconvenience and social costs caused by internal medical treatment for the aged.
Korean Patent Publication No. 10-2017-0044826 discloses a wearable device capable of measuring a bio-signal. The wearable device capable of measuring a bio-signal includes: a photoplethysmographic (PPG) signal detecting unit for detecting a PPG signal to measure a heart rate; an acceleration sensor for detecting a dynamic movement signal; a signal processing unit for amplifying the PPG signal and the dynamic movement signal and converting them into digital signals; and a wireless communication unit for processing and transferring the PPG signal and the dynamic movement signal according to the short-range wireless specification, wherein the PPG signal detecting unit, the acceleration sensor, the signal processing unit, and the wireless communication unit are mounted on a flexible substrate so that a wearer can attach or wear onto his or her body.

Korean Patent No. 10-0989694 discloses a measurement terminal for measuring a wearer's position and a bio-signal and a health control system using the same. The measurement terminal for measuring a wearer's position and a bio-signal and a health control system using the same can provide monitoring healthcare and bio-information services at all times without any restraints in time and space through communication with the health control system so that it is possible to carry out emergency relief automatically or by an emergency call by the wearer in the event of an emergency and notify the relevant medical organization for a rapid response when there is a strange symptom on the wearer.

Korean Patent Publication No. 10-2017-0012166 discloses biomedical devices for monitoring real-time medical conditions using biometric-based information communication. The invention disclosed in Korean Patent Publication No. 10-2017-0012166 relates to a method and an apparatus for forming a biometric-based information communication system. The biometric-based information communication system includes a bio-medical device having a sensing means, and the sensing means generates a biometric result. In an embodiment, the biometric-based information communication system may include a user device, such as a smart phone paired with the biometric device during communication. A biometric measurement result can trigger communication of a biometric-based information communication message.

However, because conventional arts are often formed through enumeration of rather vague concepts, the measuring device cannot measure blood pressure and blood glucose, which are the most important indicators of human body conditions, in a non-invasive manner and cannot be easily mounted on a body part to measure them simply and accurately, the conventional arts are hard to effectively support people who keenly require such wearable device and support system, such as cardiovascular patients or diabetic patients.

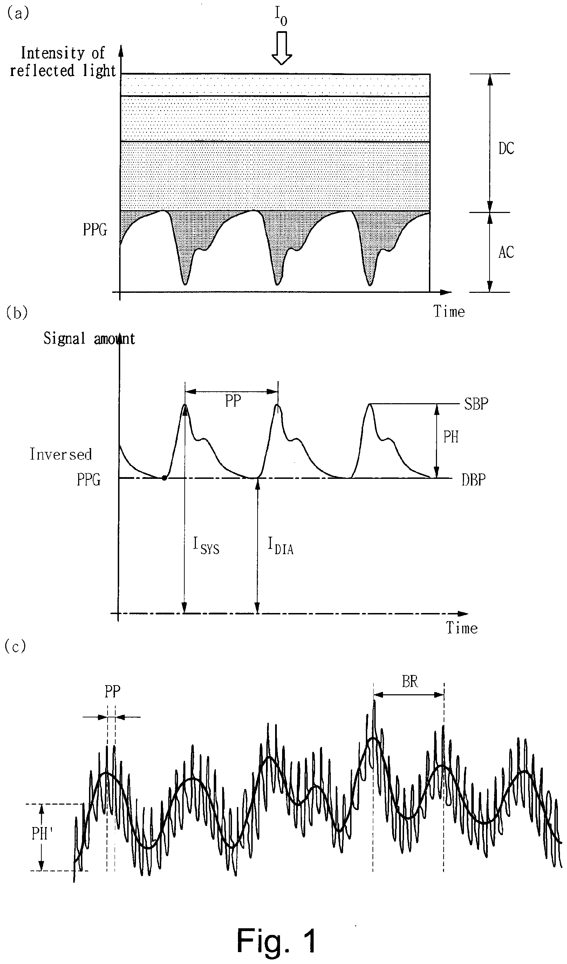

FIG. 1(a) shows a general form of a pulse wave measurable by a PPG, which is a body wearable bio-signal measuring device used widely; FIG. 1(b) shows an AC component form which is a pure blood flow change acquired by subtracting a DC component from the general form of the pulse wave; and FIG. 1(c) shows a more realistic measurement form in which a respiratory influence is included in FIG. 1(b).

The PPG is to analyse a pulse wave form at the early stage and measure a heart rate (HR) from a change in a blood flow rate, but recently, the AC component and the DC component showing the change in pulse wave can be separately reanalysed using digital signal process (DSP) technology.

While using the PPG, the AC component is a part influenced by a pulse component of arterial blood, a pulse frequency may be used as a fundamental material to measure blood pressure (BP) and can obtain information of other body conditions, for instance, vascular aging information from a second derivative waveform (SDPPG) of the AC component. The DC component is mostly influenced by skin and tissues, venous blood, non pulsatile component of arterial blood, and so on.

A photosensor assembly of the body wearable PPG generally uses green, red, and near infrared (NIR) LEDs as light sources and measures a change in reflected light intensity depending on the blood flow rate using a photodiode (PD), and the reflected light intensity is in an inverse relationship to an absorption coefficient.

In the meantime, the realistic measurement as shown in FIG. 1(c) shows a change in the overall and cyclic waveform of the DC level by breathing. Here, the general pulse wave form shows the form that a change of a short cyclic pulse form by cardiac impulse is combined with a change of a long cyclic wave component by breathing. Therefore, a breathing rate can be measured through the general pulse wave form.

The PPG includes light sources for shining light toward the blood vessels of a human body and photodiodes for measuring intensity of reflected or scattered light, and blood pressure rises or a great deal of light is absorbed by blood at the peak of the contraction phase where the blood flow rate by contraction of the heart increases, so the reflected light in the contraction phase becomes weak and reflected light in the relaxation phase becomes strong. Considering the above, an output signal in the general PPG is not the reflected light intensity but comes out in the form of an inversed PPG, so that the blood flow rate or the blood pressure level are shown as a high peak in the contraction phase and as a low valley in the relaxation phase in the form of a pulse wave. The signal amount of convention PPG is indicated based on the inversed form.

In the meantime, because blood pressure is not 0 even in the relaxation phase, a periodic amplitude change of the AC component on the base level is made.

However, the conventional blood pressure measuring device for measuring blood pressure, which includes an ECG meter and the PPG to measure blood pressure mainly using pulse transit time (PTT) and pulse wave velocity (PWV), is unsuitable or inconvenient for ambulatory BP monitoring since a user must touch the device with two hands at the same time so that the user's body forms a part of a circuit for measurement of the ECG meter when the user measures blood pressure. Finally, because the convention blood pressure measuring method does not adopt regular monitoring but adopts intermittent measurement, it is difficult to directly monitor abnormal BP variability or bio-cyclic variability.

Therefore, people still require a body wearable blood pressure measuring device which can easily and continuously measure blood pressure without a patient's action to measure.

### Disclosure:

### Technical Problem:

Accordingly, the present invention has been made in an effort to solve the above-mentioned problems occurring in the prior arts, and it is an object of the present invention to provide a human body wearable body information measuring device and a medical support system based on the same, which can measure blood pressure and blood glucose, which are important indexes to check body conditions, in a non-invasive manner, and can be easily attached to a human body and simply and accurately measure.

It is another object of the present invention to provide a human body wearable body information measuring device and a medical support system based on the same, which can continuously measure and monitor blood pressure at ordinary times without a wearer's action to measure.

It is a further object of the present invention to provide a human body wearable body information measuring device and a medical support system based on the same, which can accurately measure blood pressure and blood glucose in spite of the wearer's physical characteristics or transition elements and allow a medical organization to rapidly take proper measures in the event of an emergency, such as shock or a hurt from a fall by measuring physical movements.

### Technical Solution:

To achieve the above objects, the present invention provides a human body wearable body information measuring device including: a plurality of light sources capable of projecting lights of different wavelength ranges to a wearer's body part; a light receiving device(photodiode) for receiving lights projected from the light sources and reflected and scattered from the wearer's body part; a blood glucose analysing module, which analyses an electric signal of the light receiving device, obtains a signal amount from a signal of a pulse wave form relative to each wavelength, and calculates blood glucose concentration in blood of a subject to be measured using a differential signal amount between the signal amount obtained relative to the body of the subject to be measured and a previously inputted standard (reference signal amount); a blood pressure analysing module for analysing pulse waves of the electric signal of the light receiving device to divide the pulse waves into an incident wave and a reflected wave, and deriving a blood pressure value through a calculation process of acquiring a pulse pressure and a PRT, multiplying pulse pressure by a first coefficient, multiplying the PRT by a second coefficient, and adding the multiplied values; and a communication device which transfers measurement data of physical conditions including a blood glucose value and a blood pressure value, trends of these values of a subject to be measured obtained by the blood glucose analysing module or the blood pressure analysing module to the outside through a surrounding communication network.

In the present invention, measurement of blood pressure and blood glucose is based on the operation principle of a photo-plethysmography (PPG), and the measuring device includes the light sources, the light receiving device, and the modules. The configurations of a processor and a program to obtain a pulse wave pattern by processing an output signal of the light receiving device and process and analyse the obtained pulse wave are considerably the same as the PPG.

Moreover, the blood glucose analysing module and the blood pressure analysing module are divided from each other in order to show differences in the process and method of calculating blood glucose and blood pressure by each program substantially, and it does not mean that they have their own light sources, sensors, sensor signal processing circuits, and processors physically separated.

The device according to the present invention may be in a smart patient care (SPC) wrist band type.

The device according to the present invention may include a signal generating module and body contact terminals for ECG.

The device according to the present invention may include a thermometer for measuring the wearer's body temperature.

The device according to the present invention may include a display device for displaying at least one among numerical values of blood glucose and blood pressure of the subject to be measured obtained by the blood glucose analysing module and the blood pressure analysing module, and a changing tendency of the numerical values.

The device according to the present invention may include a 3D accelerator for sensing or measuring the wearer's physical activities to sense a hurt from a fall, and the wearer's exercise conditions, such as walking, running or others.

The device according to the present invention may include a trans-impedance amplifier (TIA), a programmable gain amplifier (PGA), and an analogue-to-digital convertor (ADC) arranged in a route for obtaining signals in order to process signals of the sensors, for instance, the light receiving device. Output signals passing through the elements (TIA, PGA, and ADC) are inputted to the processor, and the process of obtaining information for measuring body indexes, such as blood glucose and blood pressure, from the pulse waves can be carried out.

In this instance, the processor may include a digital signal processor (DSP) and a micro-controller unit (MCU), and may serve to control a controller or an LED driver which sends an LED signal to light sources (light emitting diodes). A memory and the communication device may be connected to the processor.

The device according to the present invention may further include a warning device for generating a warning signal to at least one among the wearer, a protector, and relevant organizations if at least one among the numerical values of blood glucose and blood pressure and the changing tendency expressed by the numerical values exceeds a predetermined allowable range, an allowable pattern, or an allowable rate of change.

The blood pressure calculation program forming the blood pressure analysing module according to the present invention is based on one among the following mathematical formulas or on a formula that a constant e is added to one among the following formulas:

$$BP = a*PA+b*PRT,$$

$$BP = a*PA+b*PRT+c*GI$$

$$BP = a*PA+b*PRT+d*I_{DIA}/I_{SYS}$$

$$BP = a*PA+b*PRT+c*GI+d*IR(I_{DIA}/I_{SYS}),$$

wherein proportional constants a, b, c and d and a constant e are obtained through a method of measuring blood pressure (BP) and relevant factors (PA, PRT, GI, and $I_{DIA}/I_{SYS}$ relative to subjects to be measured several times, substituting them to the above formula to deduct the most proper proportional constant value, and in this instance, the blood pressure (BP) is measured by a verified accurate blood pressure gauge, and the relevant factors are measured by the body wearable body information measuring device of the present invention.

In this instance, the proportional constants may be found through a statistic technique, such as the known multivariate statistics, and a computer simulation, and recently, the proportional constants may be deduced by reviewing the measurement values, namely, blood pressure and relevant factors, which are test data, through an artificial neural network algorithm application program obtained by adaption of such methods through machine learning or deep learning on the basis of such type of materials.

The device according to the present invention includes the plurality of light sources of near infrared wavelength range to measure blood glucose, the program of the blood glucose analysing module determines a value obtained by integration of one cycle shown by pulse waves as the signal amount when an electric signal outputted from the photodiode, which receives reflected and scattered light caused by the light investigated from each light source is shown in the form of pulse waves according to a change in blood flow rate, obtains a differential value between the signal amount and the predetermined reference signal amount, and calculates a blood glucose value based on the differential value using an associative relationship deduced between the blood glucose value and the differential value.

In this instance, the reference may be made when the blood component with blood glucose level zero is applied to a bio-tissue dummy which is similar to a human body, and the reference signal amount can be obtained by light investigation of the light sources. In order to obtain the reference signal amount, a PPG and other optical detectors which are verified to be more accurate than the measuring device of the present invention may be used.

In this instance, the plurality of light sources may use a near-infrared light with 940 nm wavelength range, a near-infrared light with 1100 nm wavelength range, a near-infrared light with 1450 nm wavelength range and a near-infrared light with 1700 nm wavelength range showing peculiar absorption peaks with respect to chemical combination in blood glucose.

As neutral light sources for correcting the pulse waves or the size of the signal amount considering light absorption level in addition to the plurality of light sources, a green light source with 530 nm wavelength range and a red light source with 660 nm wavelength range which is capable of obtaining pulse waves to measure blood pressure may be used. That is, in order to reflect reduction of red light or light in a short near infrared range by the skin colour of the subject's body part (the wrist), the device according to the present invention may have a green light source to measure reflectance of the wrist part relative to green light of which reflectance is greatly influenced by the skin colour and to reflect the measured result to correction.

The device according to the present invention on the basis of the PPG may have a pulse oximetry (PO) function to measure oxygen saturation, a red light source with 660 nm wavelength range, and a short near infrared light source with 940 nm wavelength range to accomplish the function.

In order to achieve the above objects, in another aspect of the present invention, there is a medical support system including: a communication network connected with a communication device of a body-wearable body information measuring device; an operation server connected through the communication network; and a medical organization server and support organization server connected to the network through the operation server.

For instance, the support system according to the present invention may include a body-wearable body information measuring device, a communication network connected with a communication device of the measuring device, an operation server for receiving, storing and processing body information sent by the communication device through the communication network, and a support organization such as a medical organization, fire station, a police station and so on, connected with the operation server through the communication network.

In this instance, the communication network for connecting the communication device and the operation server has at least one communication nod, and at least one among the communication nods and the measuring device can transfer unique information to the operation server so that the wearer who is wearing the measuring device can be recognized.

Meanwhile, in order to easily catch the wearer's location for emergency, the communication nod is fixed in location, and transfers unique location information of the wearer to the operation server together with the measurement data (signal) of the measuring device so as to grasp the wearer's location in real time. Alternatively, the communication nod like a personal information terminal checks the wearer's location using a geographic information system like a GPS, and transfers the location information to the operation server together with the measurement data (signal) of the measuring device so as to grasp the wearer's location in real time.

In the medical support system according to the present invention, the communication network may use various communication networks, such as the existing known internet or wireless phone communication network, besides the above-mentioned form.

The connection between the communication device of the measuring device and the communication network may be directly achieved using the communication device of the measuring device, or may be achieved by the medium of the wearer's smartphone or a personal information terminal. The communication device may include a Bluetooth device, a modem, a LAN card, or the like which can be connected with various communication networks known widely.

The network may be configured in various ways, and preferably, includes intermediate communication nods which are capable of being connected even indoors to achieve a continuous connection among the communication nods since people demand a constant connection.

The measuring device according to the present invention includes an NFC chip inside the communication device in order to transfer the wearer's unique information together with the measured state information through the communication device so that the operation server of the measuring device or a manager can check the wearer.

## Advantageous Effects:

The human body wearable body information measuring device and the medical support system based on the same according to the present invention can measure key indexes to judge body conditions, such as a breathing rate, a heart rate, blood pressure, blood glucose, oxygen saturation, and so on, conveniently, easily and continuously without any measuring action using a small-sized wearable PPG-based measuring device, monitor measured values and a change progress of the values, and can be used for proper health and disease management for a wearer.

The human body wearable body information measuring device and the medical support system based on the same according to the present invention can send the wearer's body information obtained by the measuring device to the relevant medical care institution or medical organization periodically or in real time to facilitate remote management so as to reduce cases that the wearer has to go to a hospital or a medical care institution, can reduce the number of persons, time and expenses for regular check-up or measurement and management of body indexes, and provide scientific management through continuous data so as to exactly manage patients or a person to be measured.

The human body wearable body information measuring device and the medical support system based on the same according to the present invention can easily grasp a patient's position through the communication module and the identification means of the measuring device, can rapidly get in touch with a related organization, such as a police station or a fire station, in case of emergency, for instance, when the patient gets shock or is hurt from a fall so that the related organization can rescue the patient rapidly, facilitate transport of the patient to a medical organization, and continuously provide a hospital and a medical staff with the patient's conditions during transport and before transport so that the medical staff can diagnose and treat the patient more rapidly at the same time with arrival.

In the long term, such information is collected by the medical organization or the relevant organization to form a big data and provide useful materials for medical studies and welfare policies.

## Description of Drawings:

FIGS. 1(a), (b) and (c) are graphs respectively showing a general form of a pulse wave measurable by a PPG, which is wearable on the wrist, an AC component form which is a pure blood flow change acquired by subtracting a DC component from the general form, and a form in which a respiratory influence is realistically shown on the AC component.

FIG. 2 is a schematically conceptual diagram showing a configuration of a measuring device according to a first embodiment of the present invention.

FIG. 3 is a conceptual diagram showing a PPG-based configuration of the measuring device according to a second embodiment of the present invention.

FIG. 4 is a schematic bottom side view showing the PPG-based configuration of the measuring device according to the second embodiment of the present invention.

FIG. 5 is a schematically sectional view taken along the line of A-A' of FIG. 4.

FIG. 6 is a view showing an example of a form of the peak part of the entire pulse wave on the forearm, the wrist or the finger during measurement of blood pressure and a form that the peak part is divided into an incident wave and a reflected wave.

FIG. 7 is a view showing a body infiltration level of light of each wavelength range on each body part.

FIG. 8 is a graph of two different outputs showing a changing status of a pulse wave cyclic integral signal by each frequency through a dummy and standard blood.

FIG. 9 is a graph showing differences by several frequencies between signals referring to the graph of FIG. 8 and wearer measurement signals.

FIG. 10 is a conceptual diagram showing a medical support system for supporting the wearer's health, rescue and treatment together with the measuring device.

FIGS. 11 and 12 are conceptual diagrams showing partial communication networks which can form a route for providing an operation server with a measurement data signal of the measuring device and can grasp a real-time position.

## Mode for Invention:

Hereinafter, reference will be now made in detail to the preferred embodiments of the present invention with reference to the attached drawings.

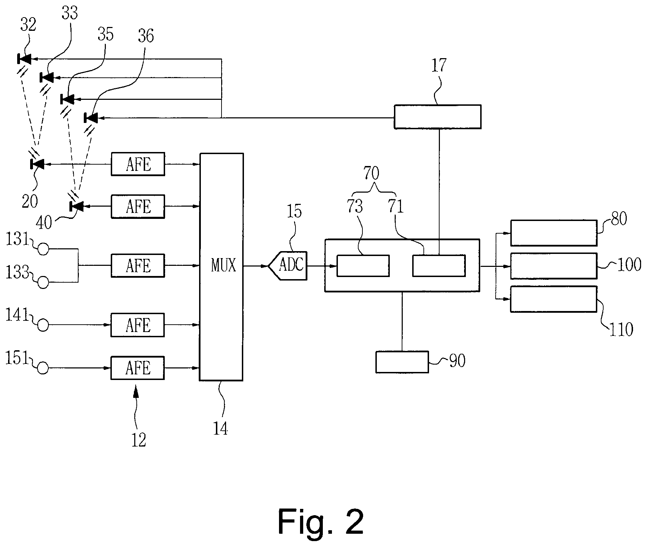

FIG. 2 is a schematically conceptual diagram showing a configuration of a measuring device according to a first embodiment of the present invention.

The measuring device of a watch form or a wristband form includes operation units, such as an optical sensor assembly having photodiodes 20 and 40 as light sources and LED light sources 32, 33, 35 and 36 to form a photo-plethysmography (PPG), ECG devices 131 and 133 for measuring a heart rate, a thermometer 141, a three-dimensional accelerator 151 for measuring a wearer's action state or movement state, a display device 100, a communication device 80, a power source 110, a memory 90, and a processor 70 for controlling them.

In general, because a terminal formed at one end of the ECG device, the optical sensor assembly, the thermometer must face the wearer's body or get in contact with the body, the terminal is mounted on the bottom surface which is a body contact surface, and another terminal of the ECG device and the display unit are mounted to be exposed on the upper surface or to get in contact with the upper surface.

Various sensors for measurement are connected to a circuitry arrangement including a plurality of elements in a main board through an analogue front end (AFE) 12. If sensor signals passing the AFE are transferred to a processor through a multiflexer (MUX) 14 and an analogue digital converter (ADC) 15. The AFE 12 and the MUX 14 are elements to effectively operate a plurality of sensors, and the analogue signal first processed and integrated through the above elements is converted into a digital signal through the ADC 15 and is transferred to the processor 70.

The processor 70 includes a digital signal processor (DSP) 73 and a micro-controller unit (MCU) 71, and a result processed through the DSP 73 is inputted to the MCU 71 and is processed by predetermined processing programs.

The processor 70 also serves to transfer a signal for operating a driver 17 or a controller to control each sensor or a trigger device related with the sensor, and also serves to operate the communication unit 80, the display unit 100 and the power source 110.

Here, the optical sensor assembly is a characteristic measurement part of the present invention, and can measure blood pressure, blood glucose, and oxygen saturation related with blood in a non-invasive manner using the PPG structure.

The ECG device, the thermometer and the 3D accelerator can measure a heartbeat signal, body temperature, physical movements or a motional state through the existing well known structure.

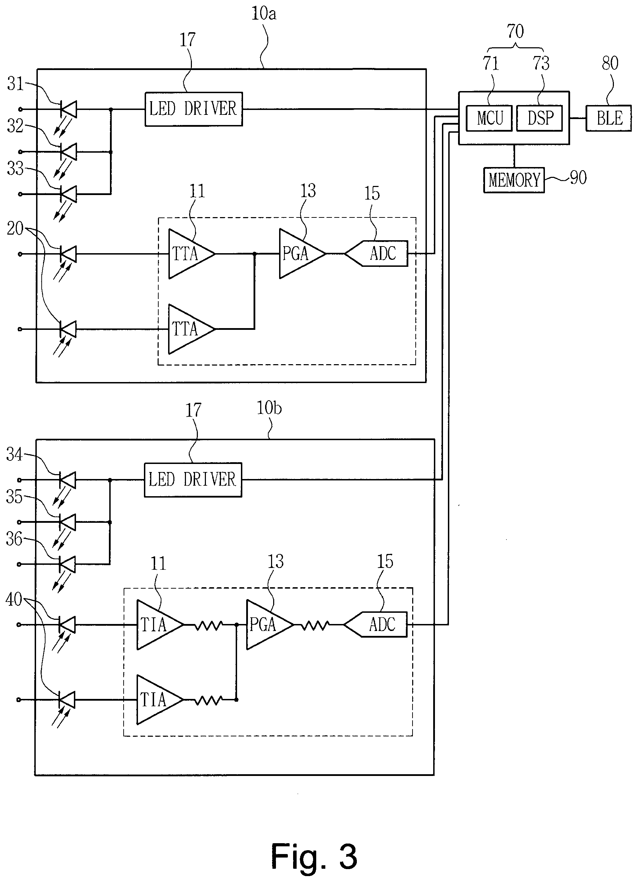

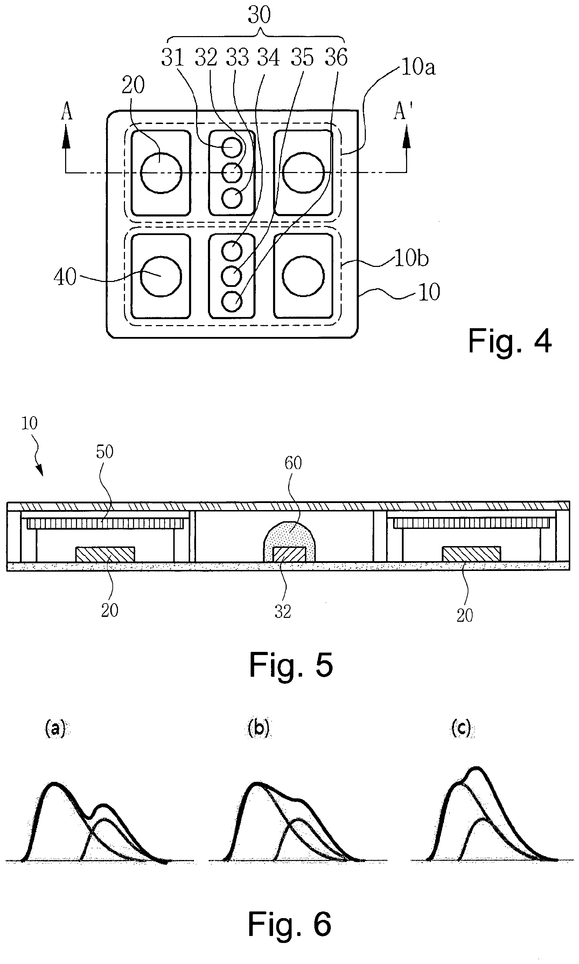

FIG. 3 is a conceptual diagram showing a PPG-based configuration of the measuring device according to a second embodiment of the present invention, and FIG. 4 is a schematic bottom side view showing the PPG-based configuration of the measuring device according to the second embodiment of the present invention.

Referring to FIG. 3, the measuring device has two sets of optical sensor assemblies 10 (10a and 10b). Each optical sensor assembly includes three LEDs as three light sources 30; an LED driver for switching the LEDs or adjusting intensity of the LEDs; two photodiodes which are photodiodes 20 and 40 for receiving scattered or reflected light and generating an electric signal after the light sources project light to a user's body part; two trans-impedance amplifiers (TIA) 11 arranged in a route for acquiring the signal in order to treat the output signal of the photodiodes; a programmable gain amplifier (PGA) 13; and an analogue-to-digital converter (ADC) 15. The first optical sensor assembly 10a located at the upper part may be a master, and the second optical sensor assembly 10b located at the lower part may be a slave.

The output signal converted after passing the elements (TIA, PGA and ADC) in each optical sensor assembly is inputted to the common processor 70, and is processed to obtain information for measuring blood pressure, oxygen saturation and blood glucose from pulse waves. The processor 70 is divided into a digital signal processor (DSP) 73 and a micro-controller unit (MCU) 71. The processor 70 serves to adjust a controller or an LED driver 17 for sending an LED signal to the LEDs, and a memory 90 and a communication unit 80 are connected to the processor.

The processor performs various actions, for instance, lighting of the light sources by prefilled program and reference data, distinguishing the output of the photodiodes, acquiring a signal amount by waveform integration by each wavelength range for calculating blood glucose, and obtaining a blood glucose value by substituting to a formula based on the size of the differential signal amount by frequencies related with blood glucose obtained through comparing the signal amount with the reference data.

Referring to FIG. 4, the optical sensor assemblies 10 are divided into two areas, are located at the upper part and a lower part on the bottom surface, and has three light sources 31, 32 and 33 located at the centre of the upper optical sensor assembly 10a, three light sources 34, 35 and 36 located at the centre of the lower optical sensor assembly 10b, and the two photodiodes 20 and 40 are mounted at the right and left sides of the light source one by one.

The upper optical sensor assembly 10a includes a green light source 31 with 530 nm wavelength range in a visible ray area, a red light source 32 with 660 nm wavelength range, and a near-infrared light source 33 with 940 nm wavelength range, wherein the light sources are mounted vertically, and the photodiodes 20 disposed at both sides use a photodiode based on a silicon (Si) wafer.

The green light source can serve as a reference light source for compensating an influence of body absorption in light intensity of different light sources since being absorbed well into the human body, and the red light source and the short near-infrared light source may be used the most appropriately to obtain an AC component and a PRT by pulse wave measurement and pulse wave analysis for measuring blood pressure.

The red light source and the short near-infrared light source may form light source sets the most appropriate for measuring oxygen saturation. That is, when the red light with 660 nm wavelength is used, a strong light absorption by oxidized haemoglobin is generated and when the short near-infrared light with 940 nm wavelength range is used, a strong light absorption by haemoglobin is generated so that we can find oxygen concentration or oxygen saturation of blood.

In the lower optical sensor assembly, the near-infrared light sources 34 and 35 with 1200 nm and 1450 nm wavelength ranges and the near-infrared light source 36 with 1700 nm wavelength range are mounted vertically, and the photodiodes 40 disposed at both sides use the photodiodes based on an indium, gallium and arsenic (InGaAs) wafer with excellent sensitivity to the rays with the wavelength ranges.

According to infrared spectroscopic data well known, with respect to near-infrared rays with wavelength ranges similar to the wavelength of the light sources, an energy band which is easily absorbable by chemical combination of ROH, AROH, CH and so on is overlapped to the 940 nm wavelength range, an energy band which is easily absorbable by chemical combination of $H_2O$, ROH, ArOH, $CH_2$, $CH_3$ and so on is overlapped to the 940 nm wavelength range, and an energy band which is easily absorbable by chemical combination of CH, $CH_2$, $CH_3$ and so on is overlapped to the 1688 nm wavelength range. Absorbance in the wavelength ranges relative to various materials including glucose has been well known by the existing infrared spectroscopy, and mutual proportion of the absorbance in the wavelength ranges is nearly uniform. In this embodiment, practical infrared light sources with wavelength ranges similar to the above wavelength ranges are prepared, and glucose concentration or the blood glucose level in blood is measured using the infrared spectroscopic data.

Of course, it is also possible to form the optical sensor assembly by adding a light source with different near-infrared wavelength range, removing some of the light sources to reduce the number of the light sources, or replacing with a light source with another wavelength range, for instance, the light source with 1450 nm wavelength range may be substituted with a light source with 1100 nm wavelength range. Such a selection is made based on the common standard that there is a light absorption range related with blood glucose to detect the blood glucose level the most distinctly. However, development easiness, such as expenses, element stability, commercialization, and so on, may be considered practically.

When the photodiodes are arranged at both sides of the light source, because sensing ability of the photodiodes may be not satisfied with the sensing ability of the existing commercial photodiodes, the number of the photodiodes is increased to accurately analyse signals.

Moreover, when the PPG type device is worn in the form of a wrist band or a watch, there may be a change in wearing position, or the position of the artery may not match the position of the light source and the photodiode. So, in order to solve the problem, the arrangement of the photodiode is made so that even one photodiode can receive the reflected light signal and the scattered light signal.

FIG. 5 is a schematically sectional view taken along the line of A-A' of FIG. 4.

A hemispherical convex lens 60 is formed above the LED used as the red light source 32 in order to raise concentration of light projected toward the body part, and a diffractive optical element lens 50 which may be a condensing lens is mounted above the photodiode, which is the photodiode 20, in order to focus light entering from the outside. The photodiode is mounted wider than the LEDs in order to increase a light receiving amount. If the convex lens 60 is mounted above the photodiode, it is difficult to make the PPG type device light, small, short and thin due to its height. So, the DOE lens 50 is mounted.

The PPG type device of the wrist band type may have additional function part besides an optical non-invasive continuous vital sign monitoring function. For instance, a digital thermometer or a 3D accelerometer may be added in order to monitor other body conditions, physical movement states, or a hurt from a fall. Through the above, the PPG type device can analyse correlation with a vital sign trend and monitor an unexpected fluctuation so as to provide the wearer and a medical officer with a warning or am attention signal.

In this embodiment shown in FIGS. 3 to 5, mainly the measuring of blood pressure, blood glucose and oxygen saturation are described based on the PPG, but as shown in FIG. 2, another measuring function part may be added, for instance, a digital thermometer or a 3D accelerometer may be added in order to monitor other body conditions, physical movement states, or a hurt from a fall. Through the above function parts, the PPG type device can analyse correlations with vital sign trends and monitor unexpected fluctuations, give a warning to the wearer through a warning device, or send the wearer's conditions to a protector or a medical officer through a built-in communication device and a communication network and provide a warning signal.

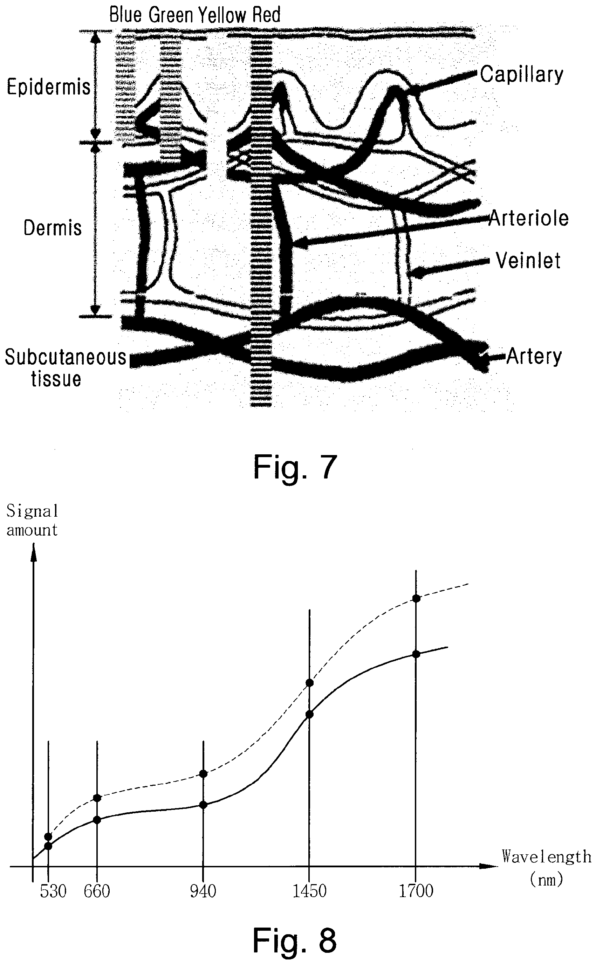

Hereinafter, referring to FIGS. 6 and 7, a method and a principle to calculate blood pressure through an analysis of a pulse wave measured by the PPG using the measuring device will be described.

(Method and principle to calculate blood pressure)

A method for measuring blood pressure through the measuring device based on the PPG will be described. First, a blood pressure measuring device is worn on the wearer's wrist, a power supply is turned on to activate the PPG type device. So, the light source projects light to the wrist continuously or in a short period of time (but to measure longer time than the pulse cycle), and measures pulse waves using the wrist blood vessel in order to obtain the pulse wave form as shown in FIG. 1(b).

Then, the light can deeply infiltrate into the human body to send a great deal of reflected light or scattered light to the photodiode. In this instance, the red light source or the short near-infrared light source, which can relatively clearly reflect a change in blood flow rate of arterial blood to the pulse wave signal amount, are used. Therefore, the configuration of the first optical sensor assembly shown in FIG. 4 can be used.

When the pulse wave is analysed by the blood pressure measuring device, time between peak and peak, a difference in signal amount between the peak showing the blood pressure raised by contraction of the heart and the valley lowered by relaxation of the heart, and time between the peak of an incident wave and the peak of a reflected wave at a measurement location can be obtained. Because the part by the DC component is not important in the pulse wave analysis, the DC component and the AC component are divided from each other and the incident wave and the reflected wave are also divided from each other to perform treatment and analysis, and then, a differential wave form of a pulse wave of a changing part may be obtained and analysed. For the above, the known various digital signal process (DSP) methods may be used.

A pulse height (PA = $I_{DIA}$-$I_{SYS}$) of a peak-to-valley among the obtained pulse waves by PPG type device is called pulse pressure, and it is the most important indicator among BP indicators. The pulse pressure is generally used as one of the most important factors for calculating blood pressure, and the blood pressure increases when the pulse pressure is high.

FIG. 6 is a view showing an example of a form of the peak part of the entire pulse wave (a complicated wave form by overlap of the incident wave and the reflected wave) on the forearm, the wrist or the finger during measurement of blood pressure and a form that the peak part is divided into an incident wave (simple wave form with great peak) and a reflected wave (simple wave form with the lowest peak). Time between the peaks of the two wave forms or pulse return time (PRT) is calculated using the incident wave and the reflected wave.

Assuming that blood pressure can be acquired using the ECG and the PTT of the PPG, the PTT and the PRT (time between incident wave peak and reflected wave peak of a compressor) greatly depend on stiffness or elasticity of blood vessels (artery), and the PPG wave form can be analysed without the ECG to calculate blood pressure in consideration of the proportional relationship between the PTT and the PRT.

If D is a distance between the heart and the blood pressure measurement position, generally the upper arm, in the blood pressure measurement using the conventional ECG, the pulse wave velocity (PWV) is the value that the distance D is divided by the PTT may be 6 m/sec in case of persons in twenties to thirties and 14 m/sec in case of persons in sixties, and the PWV increases as stiffness or hardness of the artery increases. In simple theory, PRT = 2d/PWV = 2d/D*PTT may be obtained, the PRT may be proportional to the PTT but inverse proportional to the PWV.

Based on the above possibility, data is obtained through an experiment, and people could directly check correlation between the PTT and the PRT.

If the PRT gets shorter and comes close to a third of the beat interval within a practical measurement range, a wave overlap of the incident wave and the reflected wave gets severer, so blood flow resistance gets higher and it causes an increase of blood pressure.

Therefore, in consideration of the above contents, the basic calculation formula of blood pressure may be obtained as you can see in the following formula 1 using the pulse pressure and the PRT as elementary blood pressure factor.

$$[\text{Mathematical formula 1}] \quad BP = a*PA+b*PRT$$

Moreover, in this embodiment, the first optical sensor assembly measures optical absorption by an influence of the skin colour of the wrist by operating the green light source together with the red light source or the short near-infrared light source, calculates a mutual ratio between the DC component and the AC component in the pulse wave form, and correct variability of the blood pressure value by factors influencing on the DC component(base) of the pulse wave, such as the skin, muscular tissues, blood wall, and body fluid around the wrist, through the value. Such correction can offset the influence of physical characteristics of an individual on the measurement of the PPG so as to obtain an accurate blood pressure value.

That is, the present invention is based on the PPG, light investigated to the wearer's body is absorbed, reflected or scattered at various parts of the body, and the most of the reflected and scattered light is put into the photodiode. Therefore, the light put into the photodiode may vary depending on the wearer's physical characteristics.

If the blood pressure measuring device according to the present invention is produced to measure blood pressure to be used commonly and is not personalized, the measured and calculated blood pressure value may not accurately show individual wearer since the wearer's physical characteristics are not considered.

FIG. 7 is a view showing a body infiltration level of light of each wavelength range on each body part.

Referring to FIG. 6, in order to consider individual differences, measurement which can be used as a reference showing a physical light absorption level of the individual wearer is carried out using green light absorbed into body tissues relatively well, and the measurement result is reflected to calculation of blood pressure. That is, if the light absorption level is high, absorption levels of red light or near-infrared light are also high, and the light put into the photodiode as reflected light or scattered light gets weaker and raises the (inversed) signal amount level displayed on the PPG. Therefore, it is necessary to deduct such an influence when the blood pressure value is calculated in such a way that the blood pressure value is to be lowered.

Furthermore, referring to FIG. 1, if the wearer is big and fat, there are the possibilities that the amount of light which enters the wearer's body from the light source, is reflected or scattered, and then, enters the photodiode is reduced, the DC level in the signal amount gets higher, an IR($I_{DIA}/I_{SYS}$) value also gets higher, and the measured blood pressure value is higher than the actual value. In this instance, $I_{DIA}$ is a signal value intensity in the relaxation phase, $I_{SYS}$ is a signal value intensity in the contraction phase, and IR value is a ratio between $I_{DIA}$ and $I_{SYS}$. Therefore, it is necessary to calculate the blood pressure value to be lowered by considering the DC component as the light absorption level is reflected.

When variabilities by the ratio IR($I_{DIA}/I_{SYS}$) between the DC component and the AC component due to the physical light absorption level (GI) and the physical characteristics are all reflected, the blood pressure calculation formula will be changed from the basic formula of the above-mentioned mathematical formula 1 to a mathematical formula 2 or a mathematical formula 3 obtained by adding a constant e to the right side of the mathematical formula 2.

[Mathematical formula 2]  BP = a*PA+b*PRT+c*GI+d*IR

[Mathematical formula 3]  BP = a*PA+b*PRT+c*GI+d*IR+e

In the meantime, in consideration of the above in relation with the proportional constant of the mathematical formulas, the proportional constant a will be a positive number since blood pressure gets higher if the pulse pressure is high, the proportional constant b will be a negative number since blood pressure gets higher when blood vessel stiffness is high and the PRT gets lower when blood vessel stiffness is high, and the proportional constants c and d will be negative numbers since blood pressure will be higher than the actual value if green light absorption level (IG) is high or the ratio (IR) of the DC level is high.

The above blood pressure calculation formula is internally stored in the blood pressure measuring device of the present invention in the form of programs. Therefore, if the analysis result of the pulse wave and the measurement result of green light absorption are put in the calculation formula, a blood pressure value is calculated by a predetermined proportional constant or a predetermined constant.

In this instance, the proportional constant or the constant in the calculation formula can be obtained by measuring blood pressure and relevant factors (PA, PRT, GI, and IR) to various subjects to be measured several times and substituting the factors into the calculation formula in order to derive the most proper proportional constant value. In this instance, blood pressure is measured by another precise blood pressure gauge and the relevant factors are measured by the same PPG type device as the blood pressure measuring device of the present invention, the measurement values are put into the calculation formula to make many formulas, and then, a proportional constant which can satisfy the above formulas the most proximately can be found.

The above process may be achieved through a statistical method, such as the known multivariate statistical technique, and a computer simulation, and recently, may derive the proportional constant by reviewing or studying the measurement values, namely, blood pressure and relevant factors, which are test data, through an artificial neural network algorithm application program obtained by adaption of such methods through machine learning or deep learning on the basis of such type of materials.

Through the above process, in the present invention, an idea or a paradigm to calculate blood pressure by an optical method only using the PPG is justified, and the influence on the blood pressure value of a secondary element, such as the light absorption level or the DC component when the optical method is used were removed using a proper standardization.

The artificial neural network is a logical foundation of artificial intelligence for obtaining a method to solve problems by itself through deep learning. Recently, lots of the artificial neural networks have been developed, so a detailed description of the artificial neural network will be omitted.

Of course, according to embodiments, a calculation formula (BP = a*PA+b*PRT+c*GI) that only the influence body light absorption level is included to the basic calculation formula (BP = a*PA+b*PRT) may be used in order to calculate blood pressure or a calculation formula (BP = a*PAP+b*PRT+d*$I_{DIA}$/$I_{SYS}$) may be used to reflect only the influence by the DC component of the pulse wave instead of the body light absorption level.

These calculation formulas may be stored and embodied in the measuring device in the form of a program. So, if PA, PRT, GI, IR($I_{DIA}$/$I_{SYS}$), and so on as necessary factors are measured, an embedded processor of the measuring device puts the measured value into the calculation formula to calculate blood pressure.

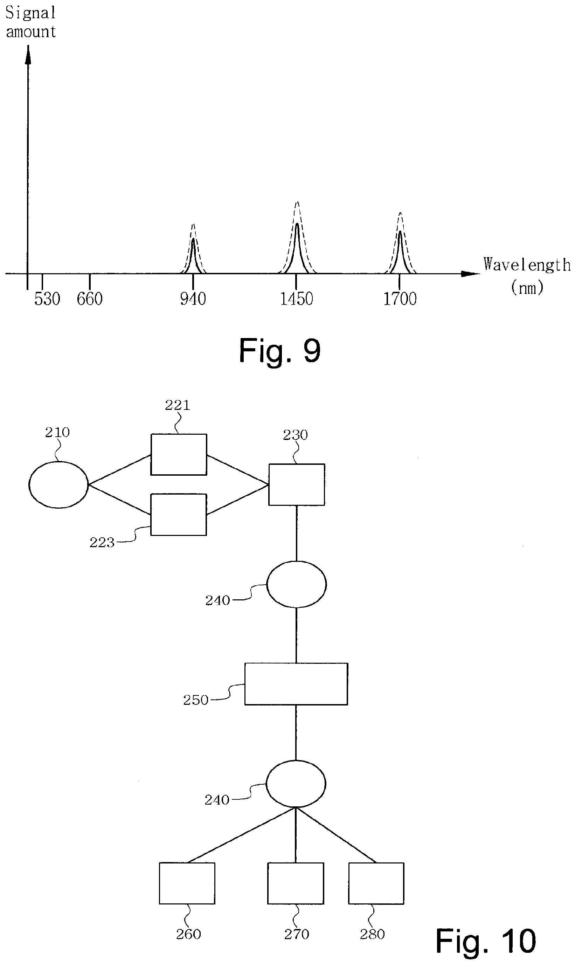

Hereinafter, referring to FIGS. 8 and 9, a method and a principle to calculate blood glucose through analysis of pulse waves measured by the PPG of the measuring device according to the present invention will be described.

[Blood glucose calculation method and principle]

The absorption level in the wavelength range of various materials including glucose has been widely known by the existing infrared spectroscopy, and mutual ratios of the absorption levels in the wavelength range are nearly uniform.

The present invention measures glucose concentration or the blood glucose level in blood using such infrared spectroscopic data. However, blood includes various materials including water, blood plasmas, and blood cells, and especially, if the PPG is used, because light getting out of the light source reaches blood after passing through the skin and other human tissues, the PPG performs infrared spectroscopy to the skin, the human tissues and the blood whenever blood glucose is measured. Therefore, in order to measure blood glucose, the basic data of the above materials must be considered, and it requires lots of efforts to measure blood glucose.

Therefore, when the present invention uses the infrared spectroscopy, for more simple analysis, the infrared spectroscopy according to the present invention does not absolutely analyse blood which is to be measured but carries out infrared spectroscopy to a subject (reference) of a base state on the basis of standard blood or reference blood from which blood glucose is removed so as to obtain a base data or a reference graph, and then, the data is previously inputted into an analysing module.

Additionally, in the present invention, infrared spectroscopy is carried out to the subject's blood including blood glucose while keeping conditions except for blood glucose of the reference subject to be the most similar, so as to obtain a subject-related data or a subject-related graph.

Here, the present invention use PPG type and projects infrared light to the human body and the photodiode receives and analyses the light reflected and scattered from the human body in order to measure the blood glucose level of the subject. During measurement, instant reflection and intensity of scattered light are changed according to a blood flow rate, and the blood flow rate is changed depending on changes of pulse waves by time.

However, because the blood glucose level is uniform even though the change of pulse waves rises, in order to obtain a stable value, the present invention does not use instant reflection and intensity of scattered light in measurement of the blood glucose level, but uses a signal amount as an integral value or an average time value obtained when an amount of instant signals, for instance, an amount of light absorption is integrated relative to one cycle on the premise that the pulse waves are stable and cyclic (hereinafter, the terms of 'signal amount' means an integral value).

First, in order to measure the standard blood from which blood glucose is removed, a subject to be measured by a glucose monitoring device is prepared. However, if the glucose monitoring device is mounted on the wrist of a human body, near infrared light investigated from the glucose monitoring device passes a route including materials influencing on the DC component, such as the skin, cutaneous tissues. So, in order to measure blood glucose in a non-invasive manner, a value (signal amount) obtained in such a way that near infrared light is projected only to the standard blood and reflected and scattered light is received and measured cannot be used as the reference value.

Therefore, ideally, it is good to obtain a reference value (signal amount) by applying the standard blood to a target part of the human body to be measured. However, because it is not practically possible to apply the standard blood to the human body, a base is formed in such a way as to make a dummy having conditions similar to the target part of the human body to be measured and apply the standard blood to the dummy, and a reference value is obtained using the base. Such a dummy may be made using a pig's body part having little subcutaneous fat, which is very similar to the human body in components. The original blood is removed and the standard blood is injected into the blood vessel of the dummy so as to create the dummy similar to the blood vessel of the human body.

In order to measure the reference value by applying the infrared spectroscopy to the dummy, near infrared light including a near infrared band to be used first is projected to the dummy, the photodiode receives reflected and scattered light, and then, data on the size of the signal amount by wavelength bands is obtained and stored by an analysing module. In this instance, since there is no change in a pulse wave form, the signal amount is obtained by an integral value which the uniform instant signal amount is simply multiplied by the pulse wave cycle (time).

The data may be expressed in the form of a numerical value of the signal amount relative to the plurality of light sources by wavelength bands to be measured by the PPG type glucose monitoring device, but preferably, may be expressed in a continuous graph curve which continues the numerical value of the signal amount relative to a continuous wave range including the wavelength band, or a subdivided wavelength band. In this instance, in order to measure the continuous wavelength band, an NIR spectrometer may be used.

The graph curve greatly depends on sensing characteristics (sensitivity by wavelength range) of the light receiving device, namely, the photodiode, and a ratio of the signal amount by the wavelength range to be measured may not form a simple linear relationship like a simple proportional relationship. In this aspect, when the NIR spectrometer is used, it is preferable that the photodiode for measurement have the same characteristics as the photodiode of the glucose monitoring device.

In order to make a reference curve (graph curve), a plurality of reference curves are made by the size of the signal amount while adjusting the signal amount obtained through a method of controlling the quantity of light of the light source of the measuring device, such as the NIR spectrometer, and then, are used to acquire the blood glucose level. When the reference curve relative to two neighbouring signal amounts is created, a reference curve between the two signal amounts may be made presumptively, and it is also possible to obtain the reference curve in all signal amount levels through the above work, which is known commonly. A statistic technique may be applied to the work, and deduction using an artificial neural network is also possible. Such a method may be achieved using an application program embedded in the analysing module in the glucose monitoring device, so necessary base data may be stored in a memory of the blood glucose analysing module.

FIG. 8 is a view showing an example of such a reference curve.

The reference curve is the sum of the signal amounts in a near infrared area by various materials of the base, and peaks of the signal amounts by the materials are all involved therein. In order to make the reference curve accurate and delicate, besides the work to measure the signal amount while changing by applying near infrared light to the dummy in the near infrared band, work to check an aspect of the peak of a signal amount change curve according to wavelength of near infrared light relative to the previously known components to check and verify whether measurement is carried out correctly may be accomplished.

Of course, the reference value may be data of a discrete value set like the signal amount value (numerical value) by wavelength range to be measured as well as the reference curve. As described above, the reference value is inputted to the memory of the blood glucose analysing module in various forms (the graph curve, the simple numerical data, or others).

When the obtained reference value is prepared as the standard for measurement, the body part of the subject to be measured is measured. For measurement, the glucose monitoring device of the present invention is used, a signal amount by each wavelength range related with the plurality of embedded light sources is obtained by the glucose monitoring device.

Moreover, a reference signal amount is subtracted from the signal amount of the subject to be measured by each wavelength range in order to calculate and obtain a differential signal amount by wavelength range.

A result of the calculation may be expressed into a peak graph like the form shown in FIG. 9.

In this instance, ideally, the signal amount forming the reference value in the wavelength range which does not relate to blood glucose is exactly the same as the signal amount measured relative to the subject to be measured and has the value of 0.

However, due to differences between the dummy and the human body in quantitative and qualitative aspects, it is difficult that the differential signal value relative to the reference light source becomes zero even though the signal value is obtained in the same measurement condition. The reference and the measuring device relative to the subject to be measured have sameness qualitatively, but if there is a difference in scale, the intensity of radiation of the light source, the intensity of the photodiode, and other may have an influence on the size of the signal amount.

Therefore, in order to obtain the differential signal amount, the signal value obtained in the same measurement condition, for instance, under the condition that the same output is applied to the light sources, with respect to the reference subject (dummy) and the subject to be measured is not used, the reference curve or the reference signal amount set having the same signal amount as the signal amount obtain as the result that the subject to be measured is measured by the glucose monitoring device of the present invention in the wavelength range of the reference light is selected so as to obtain the differential signal amount in each wavelength range related with the plurality of light sources of the glucose monitoring device, and the differential signal amount is used to calculate the blood glucose amount.

In other words, because it ignores a physical difference or a base difference between the dummy and the subject to be measured to detect the signal amounts by wavelength ranges of the dummy and the subject to be measured under the same condition using the glucose monitoring device of the present invention, derive the differential signal amounts by wavelength ranges, and calculate the blood glucose amount under the assumption that the values are all related with the blood glucose amount, there is a problem. In this instance, it is required to match the level of the signal amount using light of the wavelength range which is not related with blood glucose.

For this, in the measuring device of the present invention, a light source, for instance, a green light source, for generating light of a visible light area, in which light is easily absorbed by the base part of the human body, together with the plurality of light sources for generating lights of different near infrared areas, is mounted on a first optical sensor assembly as a reference light source. In this instance, as conceptually mentioned above, it may be considered that the base part comprehensively expresses a part influencing on the size of the signal amount as well as blood glucose.

The green light does not express particular absorption level with respect to chemical combination related with blood glucose. However, because a range of fluctuation of a reflection level or an absorption level by the materials forming the base is great, the green light may be a reference to recognize the fluctuation in signal amount by the base and correct the signal amount in each wavelength range.

For example, when a reference signal amount data in the form of the reference curve (a continuous form) or the discrete reference signal amount set (discontinuous specific values) by output of the light source while varying light intensity of the plurality of light sources or the output level applied to the light sources, a reference signal amount by output level relative to green light is obtained. Furthermore, the measuring device obtains also a signal amount value relative to the body part of the subject to be measured by green light in order to find a reference signal amount which is on par with the signal amount of the subject to be measured. Next, the measuring device find an output level for expressing the reference signal amount, and finds the reference signal amount set by wavelength range by the plurality of light sources when such an output level is applied. Additionally, the blood glucose measuring device obtains a differential signal amount set compared with the signal amount set by wavelength range measured relative to the body part of the subject to be measured.

Adjustment (selection) of the reference signal amount by wavelength range can be carried out by the process of deducting a reference signal amount relative to green light when the above-mentioned base data is deducted, and then, adopting the reference signal amount by wavelength range related with a proper reference signal amount relative to green light by using the obtained base data is carried out.

Of course, such a process may be automatically carried out through the application program embedded in the blood glucose analysing module.

After the differential signal amount by each wavelength range is obtained through the above process, blood glucose of the subject to be measured can be calculated using the size of the differential signal amount. For this, as preliminary work, an associative relationship of the differential signal amounts according to changes in blood glucose amount may be obtained through a clinical test as one of base data.

That is, if the differential signal amounts relative to blood with various blood glucose amounts are obtained and the associative relationship between the blood glucose amount and the differential signal amount is obtained by using multiple regression analysis or artificial neural network to obtain the differential signal amount, the analysing module can directly calculate the blood glucose amount.

In this instance, in order to calculate the blood glucose amount, it may be in question to select which one in the differential signal amounts to wavelength ranges. Ideally, the same blood glucose amount must be acquired for any of the differential signal amounts to wavelength ranges.

However, there is possibility that the blood glucose amounts obtained by the differential signal amounts by wavelength ranges are different from each other because the signal amount value measured relative to the subject to be measured is wrong from the start or an element besides blood glucose enters not to be suitable for measurement of the blood glucose amount. In this instance, it is difficult to calculate the blood glucose amount accurately.

Therefore, in order to measure the blood glucose amount accurately, the present invention further includes a step of verifying a ratio between the differential signal amounts by wavelength range before calculating the blood glucose amount.

Because various ratio verifications can be achieved when there are many light sources for projecting light of different near infrared wavelength ranges, it is more likely to secure accurate measurement of the blood glucose amount through intercomparison. However, in consideration of cost-effectiveness of manufacturing the devices, three or four light sources of the wavelength bands specialized for detecting blood glucose are provided here.

That is, in general, for infrared spectrometry, light sources for projecting light with continuous wavelength are used. However, in this embodiment, in order to simplify the optical structure and effectively sense and easily analyse signals, infrared light sources which are specialized for blood glucose or which can easily and stably absorb chemical combination in the blood glucose related materials are selected to measure blood glucose.

The plurality of photodiodes by wavelength which are sensitive to infrared light may be also used. Of course, it is also possible to measure the absorption level of blood of the subject to be measured with respect to light with the specific wavelength band in a time-sharing method by using the photodiodes with a single characteristic. When the light source of the first wavelength is turned on by adjustment of four light sources and the photodiodes while the analysing module is controlled by the program, it is recorded that light sensed by the photodiodes is reflected light or scattered light by the first wavelength light, and it is recorded that light sensed by the photodiodes within a predetermined period of time after second, third and fourth light sources are operated by the predetermined period of time is reflected light or scattered light by light of the corresponding wavelength band.

In order to correct an influence by surrounding light besides the light sources, a method of turning off the four light sources, measuring intensity of light sensed by the photodiode, and obtaining pure intensity of light by wavelength corrected by subtracting the intensity of the sensed light from the intensity sensed when each light source projected light may be also used.

In the meantime, a method and a principle of calculating oxygen saturation through the pulse wave analysis measured by the PPG of the measuring device according to the present invention are not described, but the method and the principle of measuring blood glucose may be substituted for the above.

However, there is a difference between the infrared wavelength range suitable for detecting blood glucose and the wavelength range of light suitable for detecting oxidized haemoglobin and haemoglobin, and instead of using the reference blood, of which blood glucose is zero, to the dummy, reference bloods having perfectly reduced haemoglobin and some oxygen saturation level are used in order to make a reference cover or reference data.

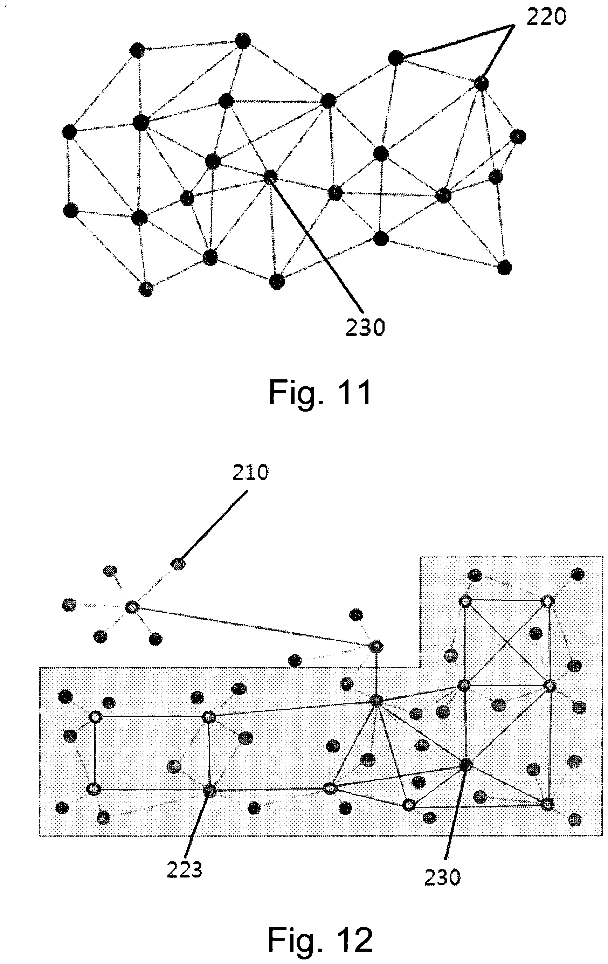

FIG. 10 is a conceptual diagram showing a medical support system for supporting the measuring device wearer's health, rescue and treatment together with the measuring device in which the communication device is included, and FIGS. 11 and 12 are conceptual diagrams showing routes or ways where the measuring device is connected through the personal digital assistants (PDA) and access points distributed with public communication networks.

Referring to the drawings, a measuring device 210 which is a wearable computer including a communication device may be connected with an operation server 250, which manages physical data and status data of the wearer received through the communication device, through a network. However, because most of people have a personal information terminal (PDA) 221, such as smart phones, the communication device is connected with the wearer's smart phone through Bluetooth communication to send and receive data or signals without overlap of functions.

The personal information terminal 221 includes an application program to be linked with the measuring device 210, receives measurement data of the measuring device whenever sensing a periodic or unusual event through Bluetooth communication, stores the measurement data received as arranged by the application program into a memory, and transfers the data to the operation server 250 through a public data (communication) network 240.

In a limited space, such as a sanatorium or a hospital, instead of the personal information terminal, fixed relay nods 223 which are densely mounted in every zone indoors and forms a Bluetooth mesh network may be used.

That is, the measuring device 210 and the operation server 250 may be connected using various public communication networks 240 and private networks. The public communication networks may be internet communication networks or mobile phone networks including wireless internet communication networks, such as wifi, and the private networks may be the Bluetooth mesh networks having the fixed relay nods 223, which has been described above, in hospitals or sanatoriums.

The personal information terminal 221 or the fixed relay nod 222 transfers the measurement data of the measuring device to a Bluetooth-wifi gateway 230, and in this embodiment, transfers the measurement data to the operation server 250 using the public internet communication network 240.

The personal information terminal, the fixed relay nod, the Bluetooth-wifi gateway and the likes may be all communication nods or communication access points for relaying signals. Such access points may simply transfer received signals, amplify or convert signals for increasing receiving sensitivity, and a plurality of the access points may be mounted and distributed in various regions in order to form a communication network. The access point (AP) may be mounted inside a big building where the public waves are hard to access, and transfer information of location of the access point together with the transferred signal so as to determine the location of the measuring device which sent data and signals to the network.

In order to transfer unique information of the measuring device or the personal information terminal and unique information on location of the access point, an RFID tag capable of sending the unique information supplementarily may be mounted, an NFC chip may be mounted, or a reader capable of receiving the information and data by being connected to a communication network may be mounted. The reader may transfer unique information related with data of the measuring device to the operation server through the communication network.

Besides the above, the communication networks and communication methods having the access points may take various forms.

For instance, as shown in FIG. 11, the plurality of Bluetooth nods 220 are connected with the neighbouring Bluetooth nods 220 in various routes like neurons of the human body to form the Bluetooth mesh, and transfer received data, namely, the data signals measured by the measuring device to a point of the corresponding area like the Bluetooth-wifi gateway 230, in the communication network.

As shown in FIG. 12, the access points like the plurality of fixed relay nods 223 are mounted around the Bluetooth-wifi gateway 230 which is a local point and if a measurement data signal is received directly from the measuring device or from the personal information terminal 221, a Bluetooth-hybrid topology in which a case that signal transfer is carried out linearly and a case that signal transfer is carried out in a plurality of routes extraordinarily are mixed between the access points. Finally, the form that all of measurement data signals are collected to the Bluetooth-wifi gateway 230 may be adopted.

Wire communication and wireless communication between the access points are all possible, and a communication method like Zigbee may be used.

The measurement data signal transferred to the local point through the signal transfer is transferred to the operation server 250 through the communication network, generally, the public communication network 240, and the operation server 250 receives the measurement data, the unique information related with the measurement data, and the location information of the access point, and stores bare data and secondary data that the bare data is processed into a database with respect to the wearer.

In the above process, an organization managing the operation server 250 compares and analyses the bare data and the secondary data with certain standard by certain analysing program, and if there is singularity in the compared and analysed result and if necessary, notifies the wearer wearing the measuring device 210, a protector or a medical organization 260 having a contact address registered for the wearer, or a public office, such as a fire station 270 or a police station 280, serving as an emergency relief centre through the public communication network 240 or a separate communication network. Of course, even though there is no singularity, the organization continuously transfers and records measurement data to the wearer's doctor or a relevant medical organization in an aspect of management. It is also possible to take necessary measures and transfer how to respond preferably together with warning.

Now, various programs for grasping and managing physical abnormality through the body information data have been developed, so a detailed description thereof will be omitted.

The support system according to the present invention can check not only body temperature, a heart rate signal form, blood pressure, oxygen saturation, blood glucose, a pulse rate and a breathing rate of the wearer at a predetermined point of time but also change forms of the values by time and trends of the values, and informs of the wearer's exercise conditions and movements by the 3D accelerator. Therefore, the support system according to the present invention can determine whether or not the wearer is in a normal state or has any problem more quickly and accurately compared with other mobile type or wearable type measuring devices, and save money and efforts caused by trial and error.

The most urgently, the support system synthetically determines the wearer's conditions by a program, which is embedded in the operation server to determine the wearer's conditions, through comparative analysis of the measurement data, such as the wearer's physical conditions, history and exercise conditions, and history data. If emergency situations, such as shock by blood glucose, such as hypoglycemic shock by a hurt from a fall or insulin, shock by hypotension or hypertension, and stroke are predicted, the support system can request a quick rescue through the public office or organization previously registered or managed in public, and provide information on registered personal matters, physical conditions, and location of the wearer to help rescue and first aid.

Moreover, if the wearer who was rescued is transported to a medical organization, the support system sends registered data, such as diagnosis, data of basic physical conditions necessary for medical treatment, and medical history, to the medical organization. Therefore, the present invention makes diagnosis and medical treatment fast and economic by removing or reducing lots of tests or examinations and saving patient-handling capacity of the medical organization.

The operation server and the organization managing the operation server may lend or sell the measuring device of the present invention to a wearer, register personal matters, and basic medical history and cautions necessary for treatment and rescue, and store and analyse various measurement data transferred from the measuring device. Therefore, the operation server and the organization managing the operation server may receive service expenses for health management or convalescence services, or public service organizations may provide health management or convalescence services.

Such organizations may evaluate the measurement data of the wearer's physical conditions continuously transferred for a long period of time, and manage the wearer's physical conditions to be in the most proper state while updating the registered matters.

If the arranged data of many people are accumulated, the accumulated data may be materials for creating new items and finding out and creating demands in relevant services for business, may be utilized as an important politic ground of policy in public and social medical and health systems, allow hypertensive patients or diabetic patients to previously take proper actions in an aspect of preventive medicine, thereby saving social convalescence, medical treatment and health expenses and providing the basic materials for making and evaluating social policies.

Furthermore, such data are results to observe many people, various diseases, and changes in state of a person who have certain medical history or took medical treatment for a long period of time, so the data may be used as materials for health-related academic research.

While the present invention has been particularly shown and described with reference to exemplary embodiments thereof, it will be understood by those of ordinary skill in the art that the above embodiments of the present invention are all exemplified and the present invention is not limited to the specific embodiments.

Therefore, it will be also understood by those of ordinary skill in the art that various changes and modifications may be made therein without departing from the technical idea and scope of the present invention and such changes and modifications belong to the claims of the present invention.

**Claims**

1. A human body wearable body information measuring device comprising:

a plurality of light sources capable of projecting lights of different wavelength ranges to a wearer's body part;
a photodiode for receiving lights projected from the light sources and reflected and scattered from the wearer's body part;
a blood glucose analysing module, which analyses an electric signal of the light receiving device (photodiode), obtains a signal amount from a signal of a pulse wave form relative to each wavelength, and calculates blood glucose concentration in blood of a subject to be measured using a differential signal amount between the signal amount obtained relative to the body of the subject to be measured and a previously inputted standard (reference signal amount);
a blood pressure analysing module for analysing pulse waves of the electric signal of the light receiving device to divide the pulse waves into an incident wave and a reflected wave, and deriving a blood pressure value through a calculation process of acquiring a pulse pressure and a PRT, multiplying pulse pressure by a first coefficient, multiplying the PRT by a second coefficient, and adding the multiplied values; and
a communication device which transfers measurement data of physical conditions including a blood glucose value and a blood pressure value, trends of these values of a subject to be measured obtained by the blood glucose analysing module or the blood pressure analysing module to the outside through a surrounding communication network.

2. The human body wearable body information measuring device according to claim 1, further comprising one among:

a signal detecting module and body contact terminals for the wearer's ECG;
a thermometer for measuring body temperature of the wearer; and
a 3D accelerator for sensing and measuring the wearer's physical movements.

3. The human body wearable body information measuring device according to claim 1, wherein a blood pressure calculation program forming the blood pressure analysing module is based on one among the following mathematical formulas or on a formula that a constant e is added to one among the following formulas:

$$BP = a*PA+b*PRT,$$

$$BP = a*PA+b*PRT+c*GI$$

$$BP = a*PA+b*PRT+d*I_{DIA}/I_{SYS}$$

$$BP = a*PA+b*PRT+c*GI+d*IR(I_{DIA}/I_{SYS}),$$

wherein a, b, c and d are proportional constants, e is a constant, a ratio $IR(I_{DIA}/I_{SYS})$ between a base component (DC component) and an AC component according to blood pressure, pulse pressure (PA), pulse return time (PRT), a physical light absorption level (GI) and physical characteristics, which are factors, relative to subjects to be measured several times is measured, they are substituted to the above formula to deduct a proportional constant value to minimize a deviation, and the blood pressure (BP) is measured by a verified accurate blood pressure gauge, and the factors are obtained using values measured by the body wearable body information measuring device.

4. The human body wearable body information measuring device according to claim 1, wherein the plurality of light sources use at least two among a near-infrared light with 940 nm wavelength range, a near-infrared light with 1100 nm wavelength range, a near-infrared light with 1450 nm wavelength range and a near-infrared light with 1700 nm wavelength range showing peculiar absorption peaks with respect to chemical combination in blood glucose in order to form the blood glucose analysing module,
wherein the program for the blood glucose analysing module determines a value obtained by integrating one cycle shown by a pulse wave as a signal amount when an electric signal outputted by the light receiving device, which receives reflected light and scattered light caused by lights from at least two of the light sources, obtains a difference value between a predetermined reference signal amount and the obtained signal amount, and calculates blood glucose based on the difference value, and
wherein the reference signal amount is obtained through a process of projecting lights controlling the quantity of light of the light sources of the existing verified near infrared spectrometer to a reference subject made by giving a blood component of a blood glucose level zero to a bio-tissue dummy which is similar to a human body, and making a plurality of reference curves or a reference data set by sizes of the signal amount obtained by projecting lights of the plurality of light sources including the at least two light sources (a value that a uniform instant signal amount is simply multiplied by a pulse wave cycle since there is no change of the pulse wave form in the reference subject) while controlling the quantity of light.

5. A medical support system comprising:

a body-wearable body information measuring device according to any one among claim 1 to claim 4;
a communication network connected with the communication device;
an operation server which receives, stores and processes body information sent by the communication device through the communication network; and
a support organization connected with the operation server through the communication network,
wherein the support organization is at least one among medical organizations, fire stations, and police stations, and
wherein the communication network for connecting the communication device and the operation server with each other includes at least one communication nod, and at least one among the communication nod and the measuring device transfers unique information of a wearer who is wearing the measuring device to the operation server.

6. The medical support system according to claim 5, wherein a location of the communication nod is fixed, and the communication nod transfers its unique location information to the operation server together with the measurement data (signal) of the measuring device so as to grasp the location of the wearer in real time.

7. The medical support system according to claim 5, wherein the communication nod is a fixed relay nod or a personal information terminal which is capable of being connected with the communication device of the measuring device, and some of the communication networks which connect the operation server with the communication device using Bluetooth communication are in the form of Bluetooth mesh or Bluetooth hybrid topology.

8. The medical support system according to claim 7, wherein the Bluetooth mesh or the Bluetooth hybrid topology is connected with a public communication network through a Bluetooth-wifi gateway.

(a)

(b)

(c)

Fig. 1

Fig. 2

Fig. 3

Fig. 4

Fig. 5

(a)          (b)          (c)

Fig. 6

Fig. 7

Fig. 8

Fig. 9

Fig. 10

Fig. 11

Fig. 12

## INTERNATIONAL SEARCH REPORT

| | International application No. |
|---|---|
| | **PCT/KR2019/002348** |

**A. CLASSIFICATION OF SUBJECT MATTER**

*A61B 5/00(2006.01)i, A61B 5/021(2006.01)i, A61B 5/145(2006.01)i, A61B 5/0402(2006.01)i, A61B 5/01(2006.01)i, A61B 5/11(2006.01)i*

According to International Patent Classification (IPC) or to both national classification and IPC

**B. FIELDS SEARCHED**

Minimum documentation searched (classification system followed by classification symbols)

A61B 5/00; A61B 5/02; A61B 5/022; G06Q 10/10; G06Q 50/22; G06Q 50/24; A61B 5/021; A61B 5/145; A61B 5/0402; A61B 5/01; A61B 5/11

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)

eKOMPASS (KIPO internal) & Keywords: biometric, measurement, a plurality of, light source, light receiving, pulse wave, signal, blood sugar, blood pressure, communication

**C. DOCUMENTS CONSIDERED TO BE RELEVANT**

| Category* | Citation of document, with indication, where appropriate, of the relevant passages | Relevant to claim No. |
|---|---|---|
| X | JP 2016-087209 A (JSR CORP.) 23 May 2016 <br> See paragraphs [29], [30], claims 1-6 and figure 1. | 1 |
| Y | | 2,4-8 |
| A | | 3 |
| Y | KR 10-2009-0099147 A (KOREA ELECTROTECHNOLOGY RESEARCH INSTITUTE) 22 September 2009 <br> See claims 2, 6 and figure 3. | 2 |
| Y | US 5222496 A (CLARKE et al.) 29 June 1993 <br> See column 7, lines 7-9. | 4 |
| Y | KR 10-2017-0063275 A (POLESTAR HEALTHCARE CO., LTD.) 08 June 2017 <br> See paragraphs [35], [50], claims 1, 8 and figure 1. | 5-8 |
| A | KR 10-2016-0119612 A (SAMSUNG ELECTRONICS CO., LTD.) 14 October 2016 <br> See the entire document. | 1-8 |

| ☐ Further documents are listed in the continuation of Box C. | ☒ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 19 JUNE 2019 (19.06.2019) | **20 JUNE 2019 (20.06.2019)** |

| Name and mailing address of the ISA/KR | Authorized officer |
|---|---|
| Korean Intellectual Property Office <br> Government Complex Daejeon Building 4, 189, Cheongsa-ro, Seo-gu, Daejeon, 35208, Republic of Korea <br> Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2019/002348**

| Patent document cited in search report | Publication date | Patent family member | Publication date |
|---|---|---|---|
| JP 2016-087209 A | 23/05/2016 | None | |
| KR 10-2009-0099147 A | 22/09/2009 | KR 10-0981137 B1 | 10/09/2010 |
| US 5222496 A | 29/06/1993 | US 5054487 A | 08/10/1991 |
| | | US 5222495 A | 29/06/1993 |
| | | US 5246004 A | 21/09/1993 |
| | | WO 94-04070 A1 | 03/03/1994 |
| KR 10-2017-0063275 A | 08/06/2017 | None | |
| KR 10-2016-0119612 A | 14/10/2016 | CN 106055084 A | 26/10/2016 |
| | | EP 3079090 A1 | 12/10/2016 |
| | | US 2016-0287142 A1 | 06/10/2016 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020170044826
- KR 100989694

- KR 1020170012166